Europäisches Patentamt
European Patent Office
Office européen des brevets

(19)

(11) Veröffentlichungsnummer: **0 023 017**
**A1**

(12)
# EUROPÄISCHE PATENTANMELDUNG

(21) Anmeldenummer: **80104106.2**

(22) Anmeldetag: **15.07.80**

(51) Int. Cl.[3]: **C 09 B 7/10**
C 07 D 333/64, C 07 D 333/74
C 07 D 333/78, C 07 C 149/20

(30) Priorität: **18.07.79 CH 6683/79**

(43) Veröffentlichungstag der Anmeldung:
**28.01.81 Patentblatt 81/4**

(84) Benannte Vertragsstaaten:
**CH DE FR GB LI**

(71) Anmelder: **CIBA-GEIGY AG**
**Patentabteilung Postfach**
**CH-4002 Basel(CH)**

(72) Erfinder: **Bühler, Niklaus, Dr.**
**Gartenweg 30**
**CH-4310 Rheinfelden(CH)**

(72) Erfinder: **Bosshard, Hans**
**Hohe Winde-Strasse 23**
**CH-4059 Basel(CH)**

(74) Vertreter: **Zumstein sen., Fritz, Dr. et al,**
**Bräuhausstrasse 4**
**D-8000 München 2(DE)**

(54) **Verfahren zur Herstellung von symmetrischen Thioindigoverbindungen oder Gemischen von symmetrischen und asymmetrischen Thioindigoverbindungen sowie Bis-Phenylthiomaleinsäurederivate und Thionaphthenone an sich.**

(57) Symmetrische Thioindigoverbindungen (I)

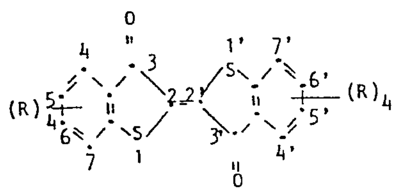
(1),

worin die R die im Patentanspruch 1 angegebene Bedeutung haben, oder Gemische von Verbindungen (I) mit asymmetrischen Thioindigoverbindungen, in denen mindestens ein R in 4- bis 7-Stellung ungleich einem Substituenten R in 4'- bis 7-'Stellung ist und die R im übrigen die im Patentanspruch 1 genannte Bedeutung haben, können nach einem neuen Verfahren auf einfache, wirtschaftliche Weise und in hoher Reinheit dadurch erhalten werden, dass man vorerst bestimmte 2,3-Dihalogenmalein- oder fumarsäurederivate, besonders 2,3-Dichlormaleinsäureanhydrid, mit einem gegebenenfalls substituierten Thiophenol oder einem Gemisch von zwei verschiedenen Thiophenolen umsetzt. Die dabei erhaltenen Bis-2,3-(Thiophenyl)-maleinsäure- oder -fumarsäurederivate werden anschliessend zu den entsprechenden 2-Thiophenylcarboxymethylen-[2H] -benzothiophen-3-onen oder Derivaten davon cyclisiert, worauf man diese Verbindungen einer weiteren Cyclisierung unterwirft.

Die erfindungsgemäss herstellbaren Thioindigoverbindungen sind zum Teil bekannt und finden insbesondere Anwendung als Küpenoder Pigmentfarbstoffe oder für den Transferdruck.

**EP 0 023 017 A1**

CIBA-GEIGY AG
Basel (Schweiz) 1-12443/ZFO/=

BEZEICHNUNG GEÄNDERT
siehe Titelseite

# Verfahren zur Herstellung von symmetrischen Thioindigoverbindungen oder Gemischen von symmetrischen und asymmetrischen Thioindigoverbindungen

Die vorliegende Erfindung betrifft ein neues Verfahren zur Herstellung von symmetrischen Thioindigoverbindungen oder Gemischen von symmetrischen und asymmetrischen Thioindigoverbindungen. Symmetrische Thioindigoverbindungen finden bekanntlich vor allem Anwendung als Küpen- oder Pigmentfarbstoffe, während Gemische von symmetrischen and asymmetrischen Thioindigoverbindungen sich besonders für den Transferdruck eignen.

Thioindigoverbindungen können nach verschiedenen Verfahren hergestellt werden, z.B. durch oxidative Kondensation von Thianaphthenonen-3, Gemischen verschiedener Thianaphthenone-3 oder deren Carbonsäuren, oder durch oxidative Kondensation von Thianaphthenonen-3 mit einem Anil eines Thianaphthenons, einem Thianaphthenchinon oder einem 2,2-Dibromthianaphthenon-3. Als Oxidationsmittel können dabei beispielsweise Kaliumcyanoferrat(III), Eisen(III)chlorid, Kupfer(II)sulfat, Natriumpolysulfide oder Kaliumdichromat verwendet werden. Die Oxidation kann auch in Gegenwart von Alkali mit Sulfonsäuren oder Sulfonsäuresalzen von aromatischen Nitroverbindungen, vor allem Nitrobenzolen, oder mit Peroxodisulfaten vorgenommen werden [vgl. z.B. US Patentschriften 2.156.032 und 2.804.464 und deutsche Patentschrift 2.504.9359]. Die Thianaphthenone-3 ihrerseits werden durch Cyclisieren von Phenylthioglykolsäuren in Gegenwart von Schwefelsäure oder Chlorsulfonsäure oder durch Ueberführen der Phenylthioglykolsäuren in die entsprechenden Säurechloride und Friedel-Crafts-Ringschluss der Säurechloride hergestellt. Diese vorbekannten Verfahren sind insofern unbefriedigend, als sowohl bei der Herstellung der Ausgangs-Thianaphthenone-3 als auch bei der Oxidation zu den Thioindigoverbindungen hohe Ueberschüsse an Cyclisierungs- und

Oxidationsmittel benötigt werden, was eine erhebliche Belastung der Abwässer zur Folge hat und/oder aufwendige Reinigungsoperationen zur Entfernung von restlichem Oxidationsmittel erforderlich macht. Gemäss einem weiteren Verfahren können Thiosalicylsäuren mit 1,2-Dichloräthylen umgesetzt werden, worauf man das Reaktionsprodukt mit Chlorsulfonsäure dehydriert [vgl. z.B. Rodd's Chemistry of Carbon Compounds, Bd. IV, Part B, 359-360 (1977)]. Dieses letztere Verfahren hat wegen der beschränkten Zugänglichkeit und der nicht ungefährlichen Synthese von substituierten Thiosalicylsäuren (Umsetzung von diazotierten substituierten Anthranilsäuren mit Thioverbindungen) nur geringe wirtschaftliche Bedeutung erlangt.

Es wurde nun ein einfaches und wirtschaftliches Verfahren gefunden, nach dem sich symmetrische Thioindigoverbindungen oder Gemische von symmetrischen und asymmetrischen Thioindigoverbindungen in hoher Reinheit, mit guten Ausbeuten und unter ökologisch günstigeren Bedingungen herstellen lassen.

Gegenstand der Erfindung ist somit ein Verfahren zur Herstellung von symmetrischen Thioindigoverbindungen der Formel Ia oder von Gemischen aus einer Verbindung der Formel Ia und einer asymmetrischen Thioindigoverbindung der Formel Ib

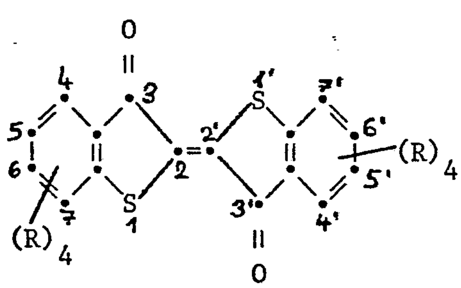

(Ia)

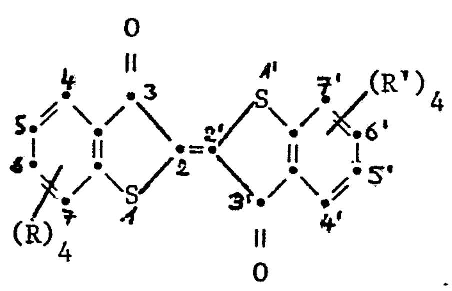

(Ib),

worin zwei R bzw. R' Wasserstoff oder Alkyl mit 1-12 C-Atomen, ein R bzw. R' Wasserstoff, ein Halogenatom oder Alkyl mit 1-12 C-Atomen und ein R bzw. R' Wasserstoff, ein Halogenatom, Alkyl, Alkoxy, Alkylthio, Alkoxyalkyl, Alkanoyloxyalkyl oder Alkylcarbamoyl mit je 1-12 C-Atomen in den Alkyl- und Alkoxyteilen und 2-13 C-Atomen in den Alkanoylteilen oder zwei benachbarte Substituenten R bzw. R'

zusammen Trimethylen oder Tetramethylen und die restlichen R bzw. R' Wasserstoff darstellen, mit der Massgabe, dass in Formel Ib mindestens ein R ungleich einem Substituenten R' ist, indem man eine Verbindung der Formel IIa oder ein Gemisch aus einer Verbindung der Formel IIa und einer Verbindung der Formel IIb

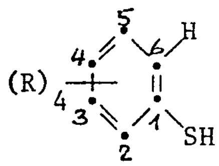

(IIa)

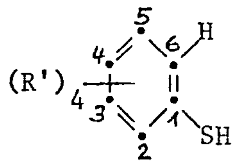

(IIb)

mit einer Verbindung der Formel III

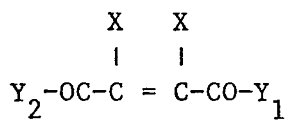

(III)

zu einer Verbindung der Formel IVa bzw. einem Gemisch aus einer Verbindung der Formel IVa und einer Verbindung der Formel IVb

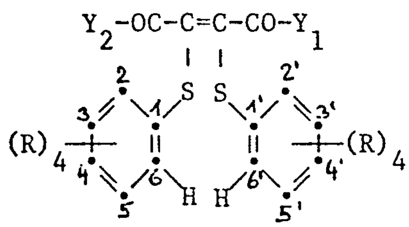

(IVa)

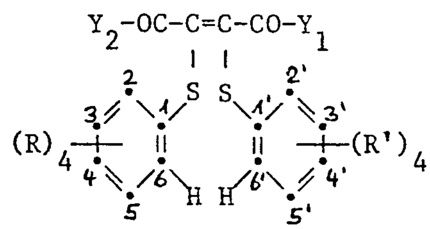

(IVb)

umsetzt, die Verbindung der Formel IVa bzw. das Gemisch aus einer Verbindung der Formel IVa und einer Verbindung der Formel IVb in Gegenwart einer Lewis-Säure in eine Verbindung der Formel Va bzw. ein Gemisch aus einer Verbindung der Formel Va und einer Verbindung der Formel Vb und/oder Vc

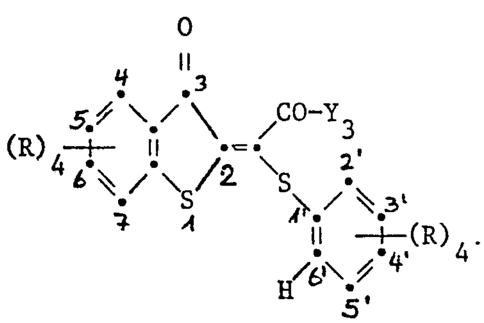

(Va)

,

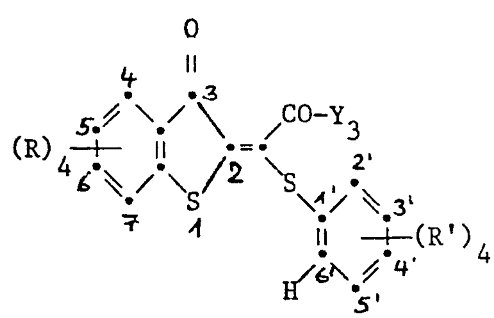

(Vb)

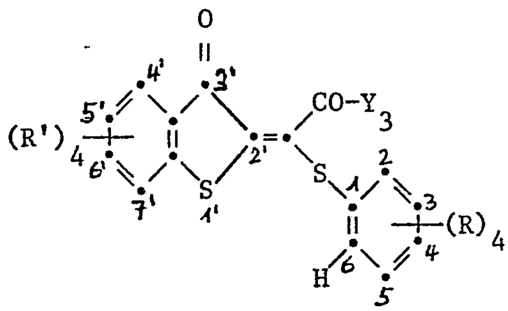

(Vc)

überführt und entweder die Verbindung der Formel Va bzw. das Gemisch aus einer Verbindung der Formel Va und einer Verbindung der Formel Vb und/oder Vc, worin $Y_3$ -OH ist, direkt durch Wasserabspaltung cyclisiert oder die Verbindung der Formel Va bzw. das Gemisch aus einer Verbindung der Formel Va und einer Verbindung der Formel Vb und/oder Vc bei $Y_3 \neq$ Chlor in die entsprechenden Säurechloride überführt und die Säurechloride in Gegenwart einer Lewis-Säure cyclisiert, wobei für R und R' das unter Formel Ia bzw. Ib Angegebene gilt, die X unabhängig voneinander Chlor, Brom oder Fluor, $Y_1$ und $Y_2$ zusammen -O- oder eines von $Y_1$ und $Y_2$ ein Halogenatom, -OH, $C_{1-6}$-Alkoxy, Phenoxy oder ein Alkalimetallkation und das andere -OH, $C_{1-6}$-Alkoxy, Phenoxy oder ein Alkalimetallkation, oder, wenn es sich bei den Verbindungen der Formel III, IVa oder IVb um ein Fumarsäurederivat handelt, auch ein Halogenatom und $Y_3$ ein Halogenatom, -OH, $C_{1-6}$-Alkoxy, Phenoxy oder ein Alkalimetallkation darstellen.

Dabei ist es überraschend, dass man die Verbindungen der Formel Ia und deren Gemische mit Verbindungen der Formel Ib dadurch aufbauen kann, dass man - entgegen den vorbekannten Verfahren - die zentrale Doppelbindung nicht in der Endstufe der Synthese durch oxidative Kondensation von zwei Thianaphthenonhälften, sondern bereits in der Anfangsstufe einführt und anschliessend die beiden Heteroringe durch Cyclisierung aufbaut. Es ist auch überraschend, dass die Cyclisierung der Verbindungen der Formel Va bis Vc nicht unter Eliminierung der Doppelbindung zur Bildung von Spiro-3,3-benzthien-3'-on-yl-2,3-dihydrobenzothiophen-2-carbonsäuren oder Derivaten davon führt, da gemäss Literaturangaben Benzoylacrylsäuren unter

wasserabspaltenden Bedingungen immer an der Doppelbindung reagieren und keine Ringschlüsse unter Beteiligung der Carboxylgruppe ergeben [vgl. z.B. G.A. Olah: Friedel Crafts and Related Reactions, Bd. III, 1, 579-81, New York 1963-65].

Die nach dem erfindungsgemässen Verfahren erhältlichen Produkte fallen im allgemeinen in analysenreiner Form an und weisen einen brillanteren Farbton als die nach den vorbekannten Verfahren hergestellten Verbindungen auf. Eine Isolierung der Zwischenprodukte der Formeln Va, Vb und Vc ist nicht unbedingt erforderlich, so dass die zweimalige Cyclisierung zu Verbindungen der Formel Ia oder deren Gemischen mit Verbindungen der Formel Ib auch im Eintopfverfahren erfolgen kann, ohne dass dadurch die Ausbeuten oder die Reinheit der erhaltenen Produkte beeinträchtigt werden.

Die Verbindungen der Formel Ia und Ib fallen fast ausschliesslich in der trans-Form an. Sie sind zum Teil bekannt. Nach dem erfindungsgemässen Verfahren lassen sich jedoch auch Verbindungen der Formel Ia oder Ib mit bisher nicht bekannten Substituentenkombinationen herstellen. Bei den Zwischenprodukten der Formeln IVa, IVb, Va, Vb und Vc handelt es sich im allgemeinen um cis/trans-Gemische. Die Zwischenprodukte der Formeln IVa, IVb und Va sind neu und ebenfalls Gegenstand der Erfindung.

Durch R, R', $Y_1$, $Y_2$ oder $Y_3$ dargestellte Alkyl-, Alkoxy-, Alkylthio-, Alkoxyalkyl-, Alkanoyloxyalkyl- oder Alkylcarbamoylgruppen können geradkettig oder verzweigt sein, sind aber bevorzugt geradkettig. Alkyl- und Alkoxygruppen R und R' sowie Alkyl- und Alkoxyteile in Substituenten R und R' weisen bevorzugt 1-8 und insbesondere 1-4 C-Atome auf, während Alkanoylteile mit Vorteil 2-9 und vor allem 2-5 C-Atome aufweisen. Als Alkoxygruppen $Y_1$, $Y_2$ und $Y_3$ werden solche mit 1-4 und besonders 1 oder 2 C-Atomen bevorzugt. Als Beispiele von definitionsgemässen Alkyl-, Alkoxy-, Alkylthio-, Alkoxyalkyl-, Alkanoyloxyalkyl- oder Alkylcarbamoylgruppen R oder R'

bzw. $Y_1$ bis $Y_3$ seien genannt: Methyl, Aethyl, n-Propyl, Isopropyl, n-Butyl, sek-Butyl, n-Pentyl, Neopentyl, n-Hexyl, n-Heptyl, 3-Heptyl, n-Octyl, n-Decyl, n-Dodecyl; Methoxy, Aethoxy, n-Propoxy, n-Butoxy, n-Hexyloxy, n-Octyloxy und n-Dodecyloxy; Methylthio, Aethylthio, n-Propylthio, Isopropylthio, n-Butylthio, n-Pentylthio, n-Hexylthio, n-Heptylthio, n-Decylthio; Methoxymethyl, 2-Methoxyäthyl, 2- oder 3-Methoxypropyl, 3-Aethoxypropyl, 2-Aethoxyäthyl, 2-n-Propoxyäthyl, 2-n-Butoxyäthyl, 2-sek-Butoxyäthyl, 4-Methoxybutyl, 4-Aethoxybutyl, 2-Octyloxyäthyl; Acetyloxymethyl,2-Acetyloxyäthyl, 2-Propionyloxyäthyl, 2-Butyroxyäthyl; Methylcarbamoyl, Aethylcarbamoyl, n-Propylcarbamoyl, Isobutylcarbamoyl, n-Butylcarbamoyl, n-Hexylcarbamoyl, n-Octylcarbamoyl, n-Nonylcarbamoyl und n-Dodecylcarbamoyl.

Stellt R oder R' eine Alkoxyalkyl- oder Alkanoyloxyalkylgruppe dar, so handelt es sich bevorzugt um Gruppen $-(CH_2)_2-O-Alkyl$ oder $-(CH_2)_2-OCO-Alkyl$ mit je 1-4 C-Atomen in den Alkylteilen.

Als Halogenatome R, R', X, $Y_1$, $Y_2$ und $Y_3$ kommen z.B. Fluor, Chlor oder Brom in Betracht. Bevorzugte Halogenatome R, R', X und $Y_1$ bis $Y_3$ sind Brom und besonders Chlor.

Stellt $Y_1$, $Y_2$ oder $Y_3$ ein Alkalimetallkation dar, so handelt es sich insbesondere um Natrium oder Kalium. Geeignete Verbindungen der Formel III sind z.B. 2,3-Dichlor- und 2,3-Dibrommaleinsäure, 2,3-Dichlor- oder 2,3-Dibromfumarsäure sowie die entsprechenden Dinatrium- und Dikaliumsalze, 2,3-Dichlormaleinsäure-monomethyl-, -monoäthyl-, -mono-n-propyl- oder-monophenylester-säurechlorid, 2,3-Dichlorfumarsäuredimethyl-,-diäthyl-, -di-n-butyl- und -diphenylester, 2,3-Dichlor- und 2,3-Dibromfumarsäuredichlorid, 2,3-Dibrom- und 2,3-Dichlormaleinsäureanhydrid. Als Verbindungen der Formel III werden solche bevorzugt, worin die X Brom und insbesondere Chlor und eines von $Y_1$ und $Y_2$ Brom und vor allem Chlor und das andere Alkoxy mit 1-4 C-Atomen, Phenoxy oder ein Alkalimetallkation darstellen. Besonders bevorzugt verwendet man als Verbindung der Formel III

Dichlormaleinsäureanhydrid. $Y_3$ in den Formeln Va bis Vc hat bevorzugt die entsprechenden Bedeutungen und stellt insbesondere -OH dar.

Als Verbindungen der Formel IIa und IIb setzt man im erfindungsgemässen Verfahren vorzugsweise solche ein, worin zwei R bzw. R' Wasserstoff oder Alkyl mit 1-4 C-Atomen, ein R bzw. R' Wasserstoff, Chlor, Brom oder Alkyl mit 1-4 C-Atomen und ein R bzw. R' Wasserstoff, Chlor, Brom,Alkyl, Alkoxy, Alkylthio, $-(CH_2)_2$-O-Alkyl, $-(CH_2)_2$-OCO-Alkyl oder -NHCO-Alkyl mit je 1-4 C-Atomen in den Alkylteilen darstellen.

Stellen in Formel IIa oder IIb zwei benachbarte Substituenten R oder R' zusammen Trimethylen oder Tetramethylen dar, so haben vorzugsweise die R bzw. R' in 3,4-Stellung die genannte Bedeutung, und die restlichen R und R' stellen Wasserstoff dar.

Bevorzugt verwendet man Verbindungen der Formel IIa und gegebenenfalls Verbindungen der Formel IIb, worin höchstens drei Substituenten R bzw. R' ungleich Wasserstoff sind. Sind drei Substituenten R bzw. R' ungleich Wasserstoff, so können diese in beliebiger Stellung an den entsprechenden Benzolkern gebunden sein.

Gemäss einer weiteren Bevorzugung setzt man Verbindungen der Formel IIa bzw. IIb ein, worin zwei R bzw. R' Wasserstoff bedeuten und die restlichen R bzw. R' ungleich Wasserstoff sind, mit der Massgabe, dass die R bzw. R' in 4- und 5-Stellung oder die R bzw. R' in 2- und 3-Stellung nicht gleichzeitig ungleich Wasserstoff sind. Besonders bevorzugt sind disubstituierte Verbindungen der Formel IIa oder IIb, worin entweder die R bzw. R' in 3- und 4-Stellung Wasserstoff und die restlichen R bzw. R' ungleich Wasserstoff sind, oder worin die R bzw. R' in 2- und 5-Stellung Wasserstoff und die restlichen R bzw. R' ungleich Wasserstoff sind.

Unter den monosubstituierten Verbindungen der Formel IIa

und IIb werden solche bevorzugt, worin das R bzw. R' in 2-, 3- oder 4-Stellung ungleich Wasserstoff ist.

In den oben erwähnten bevorzugten mono-, di- und trisubstituierten Verbindungen der Formel IIa oder IIb und den entsprechenden Zwischenprodukten der Formeln IVa, IVb, Va, Vb und Vc haben von Wasserstoff verschiedene Substituenten R oder R' mit Vorteil die oben angegebene bevorzugte Bedeutung.

Gemäss einer besonders bevorzugten Ausführungsform verwendet man im erfindungsgemässen Verfahren Verbindungen der Formel IIa, vor allem solche, worin

- die R in 2- und 5-Stellung Wasserstoff, das R in 4-Stellung Wasserstoff, Chlor, Brom oder Alkyl mit 1-4 C-Atomen, das R in 3-Stellung Wasserstoff, Alkyl oder Alkylthio mit je 1-4 C-Atomen oder die R in 3- und 4-Stellung zusammen Trimethylen oder Tetramethylen und die restlichen R Wasserstoff darstellen,
- die R in 2- und 5-Stellung Wasserstoff, das R in 3-Stellung Wasserstoff oder Alkyl mit 1-4 C-Atomen und das R in 4-Stellung -NHCO-Alkyl oder $-(CH_2)_2$-OCO-Alkyl mit je 1-4 C-Atomen in den Alkylteilen oder die R in 2- und 5-Stellung Chlor oder Brom und die R in 3- und 4-Stellung Wasserstoff bedeuten. Ganz besonders bevorzugt sind Verbindungen der Formel IIa, worin die R in 2- und 5-Stellung Wasserstoff, Chlor, Brom oder Alkyl mit 1-4 C-Atomen und die restlichen R Wasserstoff bedeuten, oder worin das R in 4-Stellung Alkyl mit 1-4 C-Atomen und die restlichen R Wasserstoff darstellen.

Die Ausgangsprodukte der Formel IIa, IIb und III sind bekannt oder können nach üblichen Methoden hergestellt werden.

Die Umsetzung der Verbindungen der Formel IIa bzw. von Gemischen aus einer Verbindung der Formel IIa und einer Verbindung der Formel IIb mit einer Verbindung der Formel III kann durch trockenes Erhitzen der Reaktionskomponenten auf etwa 50-200°C vorgenommen werden, wird aber mit Vorteil in Gegenwart eines inerten organischen Lösungsmittels und unter Zusatz einer organischen oder anorganischen Base durchgeführt.

Die Verbindung der Formel III wird zweckmässig in einer etwa 1- bis 10-fachen molaren Menge eingesetzt. Die organische oder anorganische Base wird im allgemeinen in im wesentlichen stöchiometrischen Mengen oder in einem leichten Ueberschuss (bis ca. 1,5-fache molare Menge) verwendet. Die Reaktionstemperaturen liegen im allgemeinen zwischen etwa 0 und 120°C, bevorzugt zwischen etwa 20 und 70°C.

Geeignete inerte organische Lösungsmittel für die erwähnte Umsetzung sind z.B. gegebenenfalls chlorierte aliphatische und aromatische Kohlenwasserstoffe, wie Dichlormethan, 1,2-Dichloräthan, 1,1,2,2-Tetrachloräthan, Benzol, Toluol, Chlorbenzol und Nitrobenzol; aliphatische und cyclische Aether, wie Diäthyläther, Tetrahydrofuran und Dioxan; Aethylenglykoldialkyläther mit je 1-4 C-Atomen in den Alkylteilen, wie Aethylenglykoldimethyl- und -diäthyläther; aliphatische Monocarbonsäuren mit 1-4 C-Atomen im Alkylteil und Alkylester von aliphatischen Monocarbonsäuren mit insgesamt 2-6 C-Atomen, wie Essigsäure, Propionsäure, Buttersäure, Ameisen- oder Essigsäuremethyl-, -äthyl- und -butylester; cyclische Amide, wie N-Methyl-2-pyrrolidin, N-Acetyl-2-pyrrolidon, N-Methyl-ε-caprolactam; N,N-Dialkylamide von aliphatischen Monocarbonsäuren mit 1-3 C-Atomen im Säureteil, wie N,N-Dimethylformamid, N,N-Dimethylacetamid, N,N-Diäthylacetamid und N,N-Dimethylmethoxyacetamid; Tetrahydrothiophendioxid (Sulfolan) und Dialkylsulfoxide, wie Dimethyl- oder Diäthylsulfoxid; aliphatische und cycloaliphatische Ketone, wie Aceton, Methyläthylketon, Cyclopentanon und Cyclohexanon. Bevorzugtes Lösungsmittel ist wasserfreie Essigsäure.

Beispiele von geeigneten organischen und anorganischen Basen sind tertiäre Amine, wie Triäthylamin, Pyridin, Pyridinbasen oder Alkalimetall- und Erdalkalimetallacetate. Bevorzugt sind Alkalimetallacetate, besonders Natrium- und Kaliumacetat.

Die Zwischenprodukte der Formel IVa und IVb fallen im allgemeinen in kristalliner Form an, die auf übliche Weise isoliert und gereinigt werden können, z.B. durch Umkristallisation.

Die definitionsgemässen Cyclisierungen in Gegenwart einer Lewis-Säure können in einem inerten organischen Lösungsmittel oder in der Schmelze erfolgen. Als Lewis-Säuren können z.B. eingesetzt werden: Aluminiumtrichlorid, Aluminiumtribromid, Zinkchlorid, Zinntetrachlorid, Bortrifluorid, Eisen(III)chlorid, Titantetrachlorid, Phosphortrichlorid, Phosphoroxichlorid, Antimonpentafluorid und Antimonpentachlorid. Bevorzugt verwendet man Aluminiumtrichlorid. Die Lewis-Säure wird zweckmässig im Ueberschuss eingesetzt, z.B. in einer etwa 2- bis 10-fachen molaren Menge. Geeignete organische Lösungsmittel für die genannten Cyclisierungen sind z.B.: chlorierte aliphatische oder aromatische Kohlenwasserstoffe, wie Dichlormethan, 1,2-Dichloräthan, 1,2,3-Trichlorpropan, 1,1,2,2-Tetrachloräthan und o-Dichlorbenzol; n-Pentan und n-Hexan; Nitromethan, Nitrobenzol und Schwefelkohlenstoff. Die Cyclisierungen in der Schmelze werden zweckmässig in Gegenwart von tiefschmelzenden Salzgemischen vorgenommen, z.B. in Gemischen von Aluminiumtrichlorid mit anorganischen oder organischen Salzen, wie Ammonium-, Erdalkalimetall- und Alkalimetallhalogenide, z.B. Ammonium-, Magnesium- und Calciumchlorid, besonders jedoch Natrium- und Kaliumchlorid, sowie Pyridiniumsalze, beispielsweise N-Alkylpyridiniumhalogenide. Bevorzugt für die Cyclisierungen in der Schmelze sind eutektische Salzgemische, besonders Gemische aus Aluminiumtrichlorid und Natriumchlorid und/oder Kaliumchlorid. Es können aber an sich beliebige Salzgemische eingesetzt werden, sofern damit eine ausreichende Schmelzpunkterniedrigung erzielt wird. Dabei liegen die Temperaturen zweckmässig zwischen etwa 70 und 120°C. Bei dieser Methode erübrigt sich im allgemeinen eine Zwischenisolierung von Verbindungen der Formel Va, Vb oder Vc mit $Y_3$ = -OH, weil die zweite Cyclisierung unter den Reaktionsbedingungen direkt durch Wasserabspaltung erfolgt. Werden beide Cyclisierungen in Gegenwart einer Lewis-Säure vorgenommen, so führt man diese mit

Vorteil in einem inerten organischen Lösungsmittel, wie Dichlormethan, 1,2-Dichloräthan oder 1,1,2,2-Tetrachloräthan durch. Die Temperaturen liegen dabei je nach Art des Lösungsmittels zwischen etwa 0 und 90°C. In den meisten Fällen kann die Cyclisierung in Gegenwart eines inerten organischen Lösungsmittels aber schon bei Temperaturen zwischen etwa 0 bis 40°C vorgenommen werden.

Ist $Y_3$ in den Formeln Va bis Vc ungleich Chlor, so werden die genannten Verbindungen vor der zweiten Cyclisierung in Gegenwart einer Lewis-Säure auf an sich bekannte Weise in die Säurechloride übergeführt, nötigenfalls unter vorheriger Hydrolyse von Gruppen $Y_3$=Alkoxy oder Phenoxy. Geeignete Chlorierungsmittel sind z.B. Thionylchlorid, Oxalylchlorid, Phosgen oder Phosphorpentachlorid. Die Chlorierung erfolgt gegebenenfalls in Gegenwart eines inerten organischen Lösungsmittels bei Temperaturen zwischen etwa 0 bis 100°C, vor allem etwa 0 und 80°C. Als inerte organische Lösungsmittel für die Chlorierung kommen z.B. in Betracht: N.N-Dialkylamide von aliphatischen Monocarbonsäuren mit 1-3 C-Atomen im Säureteil, wie N,N-Dimethylformamid, N,N-Dimethylacetamid oder N,N-Diäthylacetamid; chlorierte aromatische oder aliphatische Kohlenwasserstoffe, wie 1,2-Dichloräthan, Dichlormethan, Chloroform oder Chlorbenzol. Bevorzugt wird die Chlorierung in Dichloräthan mit Thionylchlorid und unter Zusatz von etwas N,N-Dimethylformamid vorgenommen.

Die Cyclisierung der Zwischenprodukte der Formeln Va, Vb und Vc durch Wasserabspaltung wird bevorzugt in Gegenwart eines wasserabspaltenden Mittels vorgenommen. Als wasserabspaltende Mittel können z.B. Polyphosphorsäure, konzentrierte Schwefelsäure, Phosphorpentoxid oder aliphatische Carbonsäureanhydride, wie Essigsäureanhydrid und Propionsäureanhydrid, verwendet werden. Die Cyclisierung kann mit oder ohne Zusatz eines inerten organischen Lösungsmittels durchgeführt werden, zweckmässig bei Temperaturen zwischen etwa 100 und 300°C, besonders etwa 120 und 270°C. Geeignete inerte organische Lösungsmittel sind z.B. gegebenenfalls chlorierte aliphatische oder

aromatische Kohlenwasserstoffe, wie 1,1,2,2-Tetrachloräthan, o-Dichlorbenzol, Trichlorbenzole, Xylole und Nitrobenzol. Die Wasserabspaltung kann aber auch durch blosses Erhitzen mit oder ohne Zusatz eines hochsiedenden organischen Lösungsmittels bei Temperaturen zwischen etwa 100 und 300°C, besonders etwa 150 und 270°C, erfolgen. Dafür geeignete Lösungsmittel sind z.B. Trichlorbenzole, Diphenyläther, 2-Chlornaphthalin und Chinolin.

Wie schon erwähnt, ist eine Isolierung und Reinigung der Zwischenprodukte der Formeln Va bis Vc nicht unbedingt erforderlich. Ist eine Isolierung erwünscht, so kann diese auf übliche Weise durch Eingiessen in ein Wasser/Eis-Gemisch oder durch Zugabe von verdünnter Mineralsäure, wie Salzsäure, Filtrieren und Waschen mit Wasser erfolgen. Auf analoge Weise können durch Cyclisierung in Gegenwart von Lewis-Säuren erhaltene Verbindungen der Formel Ia bzw. deren Gemische mit Verbindungen der Formel Ib isoliert werden.

Die nach dem erfindungsgemässen Verfahren erhaltenen Verbindungen der Formel Ia bzw. deren Gemische mit Verbindungen der Formel Ib enthalten im allgemeinen nur geringfügige Verunreinigungen. Gewünschtenfalls können sie durch Umkristallisation aus geeigneten Lösungsmitteln, wie Essigsäure, Essigsäureäthylester, Cellosolve, Aethylenglykoldimethyl- oder -diäthyläther, Aceton, Methanol oder Aethanol, oder durch Aufschlämmen in einem dieser Lösungsmittel und Abfiltrieren in analysenreine Form gebracht werden. Die Verbindungen der Formel Ia und Ib fallen in Form von roten bis dunkelvioletten Kristallen an und können auf an sich bekannte Weise als Küpen-, Dispersions- oder Pigmentfarbstoffe oder für den Transferdruck eingesetzt werden.

Die erfindungsgemäss erhaltenen Verbindungen, besonders die symmetrischen Verbindungen der Formel Ia, zeichnen sich vor allem dadurch aus, dass sie in verschiedenen Substraten, besonders Polyester- und Polyvinylchlorid, brillante, intensiv scharlachrote bis blaurote Färbungen von hoher Lichtechtheit ergeben.

Beispiele 1-6: Herstellung von Verbindungen der Formel IVa

183,6 g (1,1 Mol) 2,3-Dichlormaleinsäureanhydrid werden mit 170 g (2 Mol) wasserfreiem Natriumacetat in 1000 ml Essigsäure vorgelegt und innerhalb von 5 Minuten mit 358 g (2 Mol) 2,5-Dichlorthiophenol versetzt, wobei die Innentemperatur auf 50°C steigt. Nach 2 1/2-stündigem Erwärmen auf 85°C heizt man während einer Stunde auf 100°C auf und filtriert dann heiss von ungelösten Bestandteilen ab. Beim Abkühlen kristallisiert Bis-2,3-(2,5-dichlorthiophenyl)-maleinsäureanhydrid in orangeroten Kristallen aus; Smp. 126-128°C (392,7 g = 96,7 % d.Th.). Umkristallisieren aus heisser Essigsäure hebt den Schmelzpunkt auf 129-133°C an. (Bsp. Nr. 1).

Analyse für $C_{16}H_6Cl_4S_2O_3$:

| | | | | |
|---|---|---|---|---|
| berechnet | 42,50% | H 1,34% | Cl 31,36% | S 14,18% |
| gefunden | 42,57% | H 1,40% | Cl 31,17% | S 14,29%. |

IR-Spektrum (KBr/$cm^{-1}$, w=schwach, m=mittel, s=stark):

$1820^m$, $1755^s$, $1510^s$, $1460^s$, $1380^m$, $1255^s$, $1170^s$, $1100^s$, $1030^s$, $935^s$, $905^m$, $815^s$, $730^s$, $716^s$.

Auf analoge Weise werden hergestellt:

- Bis-2,3-Thiophenyl-maleinsäureanhydrid (Smp. 74-76°C), ausgehend von 2,3-Dichlormaleinsäureanhydrid und Thiophenol (Beispiel Nr. 2);
- Bis-2,3-(4-chlorthiophenyl)-maleinsäureanhydrid (Smp. 156-160°C), ausgehend von 2,3-Dichlormaleinsäureanhydrid und 4-Chlorthiophenol (Beispiel Nr. 3);
- Bis-2,3-(4-methylthiophenyl)-maleinsäureanhydrid (Smp. 157-160°C), ausgehend von 2,3-Dichlormaleinsäureanhydrid und 4-Methylthiophenol (Beispiel Nr. 4);
- Bis-2,3-(4-isopropylthiophenyl)-maleinsäureanhydrid (rotes Oel), ausgehend von 2,3-Dichlormaleinsäureanhydrid und 4-Isopropylthiophenol (Beispiel Nr. 5);
- Bis-2,3-(2,5-dimethylthiophenyl)-maleinsäureanhydrid (Smp. 89-91°C), ausgehend von 2,3-Dichlormaleinsäureanhydrid und 2,5-Dimethylthiophenol (Beispiel Nr. 6).

Beispiele 7-11: Herstellung von Verbindungen der Formel Va

100 g (0,22 Mol) Bis-2,3-(2,5-dichlorthiophenyl)-maleinsäureanhydrid werden bei 20°C unter Kühlung mit einem externen Eisbad langsam zu einer Suspension von 88,8 g (0,66 Mol) wasserfreiem Aluminiumtrichlorid in 190 ml 1,2-Dichloräthan gegeben und anschliessend 20 Stunden bei 20-25°C gerührt. Dann wird die erhaltene schwarze Suspension auf Eis gegossen, 30 Minuten verrührt, und das ausgefallene gelbe Produkt wird abfiltriert und mit 150 ml Essigsäureäthylester gewaschen. Nach dem Trocknen bei 60°C/13000 Pa erhält man gelbe Kristalle von 2-(2,5-Dichlorthiophenyl)-carboxymethylen-4,7-dichlor-[2H]-benzothiophen-3-on (Smp. 222-225°C, Zersetzung) in quantitiver Ausbeute (Beispiel Nr. 7).

IR-Spektrum (KBr/$cm^{-1}$ , w=schwach, m=mittel, s=stark):

$3500^{s}$ (breit), $1770^{m}$, $1690^{s}$, $1590^{s}$, $1550^{m}$, $1465^{s}$, $1360^{w}$, $1300^{w}$, $1245^{m}$, $1220^{s}$, $1190^{w}$, $1140^{w}$, $1115^{m}$, $1100^{w}$, $1040^{w}$.

Auf analoge Weise werden hergestellt:

- 2-Thiophenyl-carboxymethylen-[2H]-benzothiophen-3-on (Smp. 169°C, Zersetzung),ausgehend von Bis-2,3-thiophenylmaleinsäureanhydrid (Beispiel Nr. 8);
- 2-(4-Chlorthiophenyl)-carboxymethylen-5-chlor-[2H]-benzothiophen-3-on (Smp. 171-173°C, Zersetzung), ausgehend von Bis-2,4-(4-chlorthiophenyl)-maleinsäureanhydrid (Beispiel Nr. 9);
- 2-(4-Methylthiophenyl)-carboxymethylen-5-methyl-[2H]-benzothiophen-3-on (Smp. 182-185°C, Zersetzung), ausgehend von Bis-2,3-(4-methylthiophenyl)-maleinsäureanhydrid (Beispiel Nr. 10);
- 2-(4-Isopropylthiophenyl)-carboxymethylen-5-isopropyl-[2H]-benzothiophen-3-on (rotes Oel), ausgehend von Bis-2,3-(4-isopropylthiophenyl)-maleinsäureanhydrid (Beispiel Nr. 11).

Beispiel 12: 30 g (0,066 Mol) Bis-2,3-(2,5-dichlorthiophenyl)-maleinsäureanhydrid werden mit 88 g (0,66 Mol) Aluminiumtrichlorid, 16 g (0,28 Mol) Natriumchlorid und 10 g (0,134 Mol) Kaliumchlorid gemischt und bei 110°C zusammengeschmolzen. Dann rührt man 15 Stunden bei

130°C und giesst die schwarze Schmelze langsam auf 750 g Eis. Die violette Suspension wird abfiltriert und mit Wasser und Aceton gewaschen. Nach zweimaligem Auswaschen mit je 250 ml Aethylenglykolmonomethyläther und anschliessendem Trocknen fallen 20,4 g 4,7,4',7'-Tetrachlorthioindigo in Form blaustichig bordeaux-roter Kristalle an; Smp. > 250°C; 70,8% d.Th.

Analyse für $C_{16}H_4Cl_4S_2O_2$:

berechnet C 44,27% H 0,93% S 14,77% Cl 32,67%

gefunden C 44,2% H 0,9% S 14,7% Cl 32,1%.

IR-Spektrum (KBr/$cm^{-1}$, w=schwach, m=mittel, s=stark):

$1710^s$, $1670^s$, $1580^s$, $1505^m$, $1445^s$, $1295^m$, $1245^s$, $1190^m$, $1130^s$, $1085^s$, $1075^s$, $915^m$.

Beispiel 13: 15 g (0,033 Mol) 2-(2,5-Dichlorthiophenyl)-carboxymethylen-4,7-dichlor-[2H]-benzothiophen-3-on werden in 130 ml 1,2-Dichloräthan gelöst, mit 11,8 g (0,099 Mol) Thionylchlorid und 1,3 ml N,N-Dimethylformamid versetzt und 22,5 Stunden bei 60°C gerührt. Die rote Lösung wird am Wasserstrahlvakuum eingedampft, und die dabei anfallenden roten Kristalle des Säurechlorids werden in eine Suspension von 22,0 g (0,165 Mol) wasserfreiem Aluminiumtrichlorid in 70 ml 1,2-Dichloräthan eingetragen. Die entstehende schwarze Suspension wird 5 Stunden auf 40°C und anschliessend 15 Stunden auf 60°C erwärmt. Dann giesst man die Suspension auf 300 g Eis und filtriert 11,1 g rotbraune Kristalle ab. Diese werden getrocknet und zweimal mit je 100 ml N,N-Dimethylformamid ausgekocht. Man erhält 8,5 g (59,4% d.Th.) weinrote Kristalle des 4,7,4',7'-Tetrachlorthioindigos, dessen analytische Daten mit denjenigen der gemäss Beispiel 12 erhaltenen Verbindung übereinstimmen.

Verwendet man unter sonst gleichen Reaktionsbedingungen nur 13,2 g (0,099 Mol) wasserfreies Aluminiumtrichlorid, so erhält man 6,8 g (46,3% d.Th.) 4,7,4',7'-Tetrachlorthioindigo, das mit dem gemäss Beispiel 12 identisch ist.

Beispiel 14: 4,7,4',7',-Tetrachlorthioindigo kann auch dadurch erhalten werden, dass man aus 80 g (0,17 Mol) 2-(2,5-Dichlorthiophenyl)-carboxymethylen-4,7-dichlor-[2H]-benzothiophen-3-on auf die in Beispiel 13 beschriebene Weise das Säurechlorid herstellt und dieses anschliessend in eine Suspension von 400 g (2,98 Mol) wasserfreiem Aluminiumtrichlorid in 400 ml 1,2-Dichloräthan einträgt und 3,5 Stunden bei 40°C rührt. Nach analogem Aufarbeiten wie in Beispiel 13 beschrieben, erhält man 65,8 g (89,3% d.Th.) 4,7,4',7'-Tetrachlorthioindigo, dessen analytische Daten mit denjenigen der nach Beispiel 12 hergestellten Verbindung identisch sind.

Beispiel 15-19: Nach den in den vorangegangenen Beispielen beschriebenen Verfahren werden die in der folgenden Tabelle angegebenen Verbindungen der Formel Ia

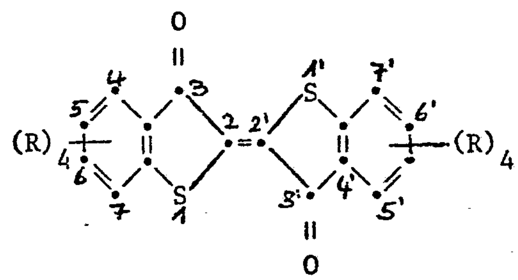

hergestellt.

Tabelle

| Beispiel Nr. | Ausgangsmaterial gemäss Beispiel Nr. | Verbindung der Formel Ia | Smp. °C | Farbe/Substrat | hergestellt gemäss Beispiel Nr. |
|---|---|---|---|---|---|
| 15 | 8 | Thioindigo | >250 | brillantes Scharlachrot/Polyester 1) | 14 |
| 16 | 9 | 5,5'-Dichlorthioindigo | >250 | blaurot/Polyvinylchlorid 2) | 14 |
| 17 | 10 | 5,5'-Dimethylthioindigo | >250 | rotviolett/Polyester 1) | 14 |
| 18 | 11 | 5,5'-Diisopropylthioindigo | >250 | bordeaux/Polyester 1) | 14 |
| 19 | 6 | 4,7,4',7'-Tetramethylthioindigo | >250 | bordeaux/Polyester 1) | 12 |

1) Polyesterfasern gefärbt gemäss Verwendungsbeispiel A

2) Polyvinylchlorid gefärbt gemäss Verwendungsbeispiel B.

Verwendungsbeispiele

Beispiel A: Ein zur Faserherstellung geeignetes unmattiertes Polyäthylenterephthalat-Granulat wird mit 1 Gew.% des gemäss Beispiel 18 hergestellten 5,5'-Dimethylthioindigo auf einer Schüttelmaschine 15 Minuten geschüttelt. Hierauf werden die Granulatkörper auf einer Schmelzspinnanlage (285°C ± 3°C, Verweilzeit in der Spinnmaschine ca. 5 Minuten) zu Fäden versponnen, die auf einer Streckzwirnanlage verstreckt und aufgespult werden. Man erhält eine brillante, rotviolette Färbung, die sich vor allem durch eine hohe Lichtechtheit auszeichnet.

Beispiel B: 65 Gew. Teile stabilisiertes Polyvinylchlorid, 35 Gew. Teile Dioctylphthalat und 0,2 Gew.Teile des gemäss Beispiel 12 erhaltenen Pigmentfarbstoffes werden miteinander verrührt und dann auf einem Zweiwalzenkalander während 7 Minuten bei 140°C hin- und hergewalzt. Man erhält eine violette Färbung von guter Licht- und Migrationsechtheit.

Beispiel C: 100 Gew. Teile Polyäthylenterephthalatgranulat und 0,2 Gew. Teile des nach Beispiel 16 hergestellten Pigmentfarbstoffes werden in einer Mischtrommel intensiv vermischt und danach während ca. 24 Stunden bei 100°C am Vakuum bei $135x10^{-3}$ Pa getrocknet. Das so behandelte Granulat wird bei ca. 280°C in einem Einwellenextruder zu einem Band extrudiert. Man erhält ein stark violett gefärbtes Band mit ausgezeichneten Lichtechtheiten.

Patentansprüche

1. Ein Verfahren zur Herstellung einer Verbindung der Formel Ia oder eines Gemisches aus einer Verbindung der Formel Ia und einer Verbindung der Formel Ib

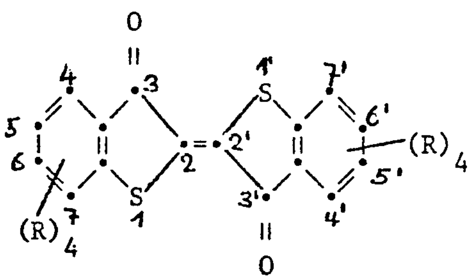

(Ia)

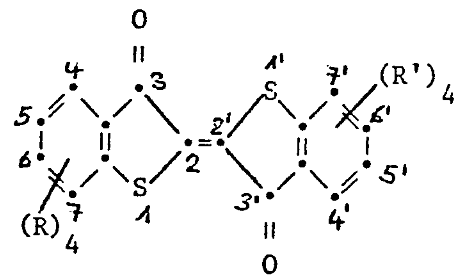

(Ib),

worin zwei R bzw. R' Wasserstoff oder Alkyl mit 1-12 C-Atomen, ein R bzw. R' Wasserstoff, ein Halogenatom oder Alkyl mit 1-12 C-Atomen und ein R bzw. R' Wasserstoff, ein Halogenatom, Alkyl, Alkoxy, Alkylthio, Alkoxyalkyl, Alkanoyloxyalkyl oder Alkylcarbamoyl mit je 1-12 C-Atomen in den Alkyl- und Alkoxyteilen und 2-13 C-Atomen in den Alkanoylteilen oder zwei benachbarte Substituenten R bzw. R' zusammen Trimethylen oder Tetramethylen und die restlichen R bzw. R' Wasserstoff darstellen, mit der Massgabe, dass in Formel Ib mindestens ein R ungleich einem Substituenten R' ist, dadurch gekennzeichnet, dass man eine Verbindung der Formel IIa oder ein Gemisch aus einer Verbindung der Formel IIa und einer Verbindung der Formel IIb

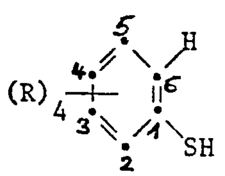

(IIa)

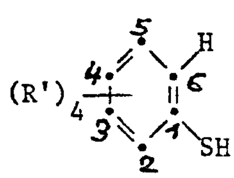

(IIb)

mit einer Verbindung der Formel III

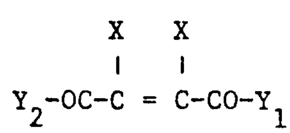

(III)

zu einer Verbindung der Formel IVa bzw. einem Gemisch aus einer Verbindung der Formel IVa und einer Verbindung der Formel IVb

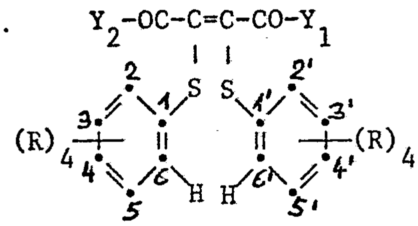

(IVa)

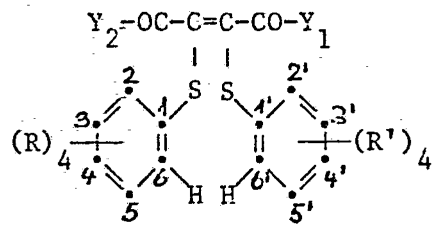

(IVb)

umsetzt, die Verbindung der Formel IVa bzw. das Gemisch aus einer Verbindung der Formel IVa und einer Verbindung der Formel IVb in Gegenwart einer Lewis-Säure in eine Verbindung der Formel Va bzw. ein Gemisch aus einer Verbindung der Formel Va und einer Verbindung der Formel Vb und/oder Vc

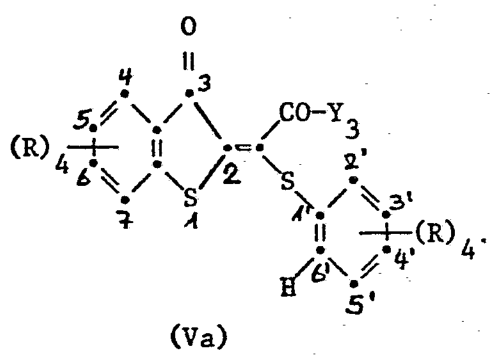

(Va)

,

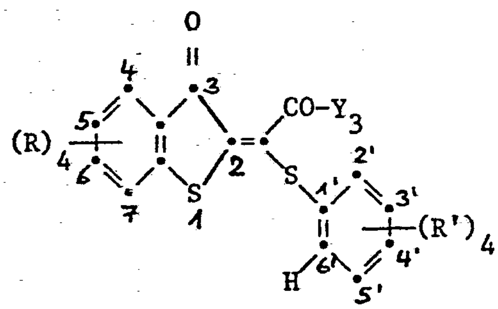

(Vb)

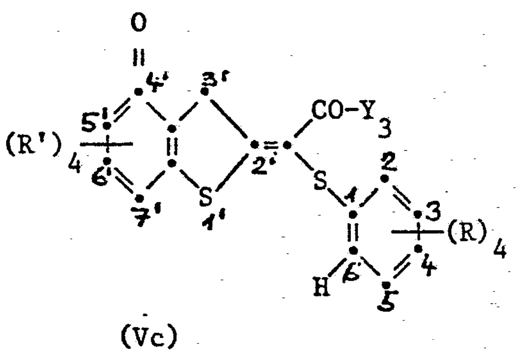

(Vc)

überführt und entweder die Verbindung der Formel Va bzw. das Gemisch aus einer Verbindung der Formel Va und einer Verbindung der Formel Vb und/oder Vc, worin $Y_3$ -OH ist, direkt durch Wasserabspaltung cyclisiert oder die Verbindung der Formel Va bzw. das Gemisch aus einer Verbindung der Formel Va und einer Verbindung der Formel Vb und/oder Vc bei $Y_3 \neq$ Chlor in die entsprechenden Säurechloride überführt und die Säurechloride in Gegenwart einer Lewis-Säure cyclisiert, wobei für R und R' das unter Formel Ia bzw. Ib Angegebene

gilt, die X unabhängig voneinander Chlor, Brom oder Fluor, $Y_1$ und $Y_2$ zusammen -O- oder eines von $Y_1$ und $Y_2$ ein Halogenatom, -OH, $C_{1-6}$-Alkoxy, Phenoxy oder ein Alkalimetallkation und das andere -OH, $C_{1-6}$-Alkoxy, Phenoxy oder ein Alkalimetallkation, oder, wenn es sich bei den Verbindungen der Formel III, IVa oder IVb um ein Fumarsäurederivat handelt, auch ein Halogenatom und $Y_3$ ein Halogenatom, -OH, $C_{1-6}$-Alkoxy, Phenoxy oder ein Alkalimetallkation darstellen.

2. Ein Verfahren nach Anspruch 1, dadurch gekennzeichnet, dass man eine Verbindung der Formel IIa oder ein Gemisch aus einer Verbindung der Formel IIa und einer Verbindung der Formel IIb verwendet, worin zwei R bzw. R' Wasserstoff oder Alkyl mit 1-4 C-Atomen, ein R bzw. R' Wasserstoff, Chlor, Brom oder Alkyl mit 1-4 C-Atomen und ein R bzw. R' Wasserstoff, Chlor, Brom, Alkyl, Alkoxy, Alkylthio, $-(CH_2)_2-O-Alkyl$, $-(CH_2)_2-O-C(=O)-Alkyl$ oder -NHCO-Alkyl mit je 1-4 C-Atomen in den Alkylteilen bedeuten.

3. Ein Verfahren nach Anspruch 1, dadurch gekennzeichnet, dass man eine Verbindung der Formel IIa verwendet.

4. Ein Verfahren nach Anspruch 1, dadurch gekennzeichnet, dass man eine Verbindung der Formel IIa verwendet, worin die R in 2- und 5-Stellung Wasserstoff, das R in 3-Stellung Wasserstoff, Alkyl oder Alkylthio mit je 1-4 C-Atomen und das R in 4-Stellung Wasserstoff, Chlor, Brom oder Alkyl mit 1-4 C-Atomen oder die R in 3- und 4-Stellung zusammen Trimethylen oder Tetramethylen und die restlichen R Wasserstoff bedeuten.

5. Ein Verfahren nach Anspruch 1, dadurch gekennzeichnet, dass man eine Verbindung der Formel IIa verwendet, worin die R in 2- und 5-Stellung Wasserstoff, das R in 3-Stellung Wasserstoff oder Alkyl mit 1-4 C-Atomen und das R in 4-Stellung -NHCO-Alkyl oder $-(CH_2)_2-OCO-$Alkyl mit je 1-4 C-Atomen in den Alkylteilen bedeuten oder die R in

2- und 5-Stellung Chlor oder Brom und die R in 3- und 4-Stellung Wasserstoff bedeuten.

6. Ein Verfahren nach Anspruch 1, dadurch gekennzeichnet, dass man eine Verbindung der Formel IIa verwendet, worin die R in 2- und 5-Stellung Wasserstoff, Chlor, Brom oder Alkyl mit 1-4 C-Atomen und die restlichen R Wasserstoff bedeuten, oder worin das R in 4-Stellung Alkyl mit 1-4 C-Atomen und die restlichen R Wasserstoff bedeuten.

7. Ein Verfahren nach Anspruch 1, dadurch gekennzeichnet, dass man als Verbindung der Formel III Dichlormaleinsäureanhydrid verwendet.

8. Ein Verfahren nach Anspruch 1, dadurch gekennzeichnet, dass man die Umsetzung einer Verbindung der Formel IIa oder eines Gemisches einer Verbindung der Formel IIa und einer Verbindung der Formel IIb mit einer Verbindung der Formel III in Gegenwart eines inerten organischen Lösungsmittels und unter Zusatz einer anorganischen oder organischen Base bei einer Temperatur zwischen etwa 0 und 120°C vornimmt.

9. Ein Verfahren nach Anspruch 1, dadurch gekennzeichnet, dass man die Cyclisierungen in Gegenwart einer Lewis-Säure in Gegenwart eines inerten organischen Lösungsmittels bei einer Temperatur zwischen etwa 0 und 90°C oder in der Schmelze unter Zusatz von Natrium- und/oder Kaliumchlorid bei einer Temperatur zwischen etwa 70 und 120°C vornimmt.

10. Ein Verfahren nach Anspruch 1, dadurch gekennzeichnet, dass man als Lewis-Säure Aluminiumtrichlorid verwendet.

11. Eine Verbindung der Formel IVa oder IVb

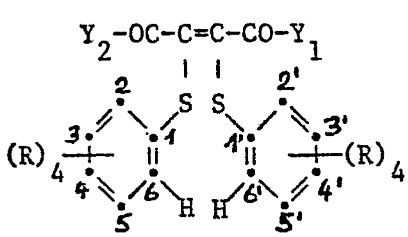

(IVa)

oder

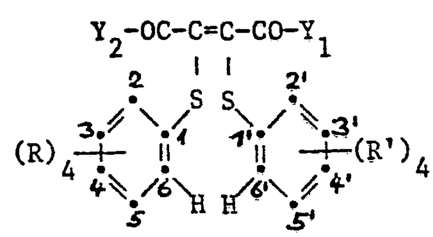

(IVb),

BAD ORIGINAL

worin zwei R bzw. R' Wasserstoff oder Alkyl mit 1-12 C-Atomen, ein R bzw. R' Wasserstoff, ein Halogenatom oder Alkyl mit 1-12 C-Atomen und ein R bzw. R' Wasserstoff, ein Halogenatom, Alkyl, Alkoxy, Alkylthio, Alkoxyalkyl, Alkanoyloxyalkyl oder Alkylcarbamoyl mit je 1-12 C-Atomen in den Alkyl- und Alkoxyteilen und 2-13 C-Atomen in den Alkanoylteilen oder zwei benachbarte Substituenten R bzw. R' zusammen Trimethylen oder Tetramethylen und die restlichen R bzw. R' Wasserstoff darstellen, mit der Massgabe, dass in Formel IVb mindestens ein R ungleich einem Sutstituenten R' ist, $Y_1$ und $Y_2$ zusammen -O- oder eines von $Y_1$ und $Y_2$ ein Halogenatom, -OH, $C_{1-6}$-Alkoxy, Phenoxy oder ein Alkalimetallkation und das andere -OH, $-C_{1-6}$-Alkoxy, Phenoxy oder ein Alkalimetallkation oder, wenn es sich bei den Verbindungen der Formel IVa oder IVb um ein Fumarsäurederivat handelt, auch ein Halogenatom bedeutet.

12. Eine Verbindung der Formel Va

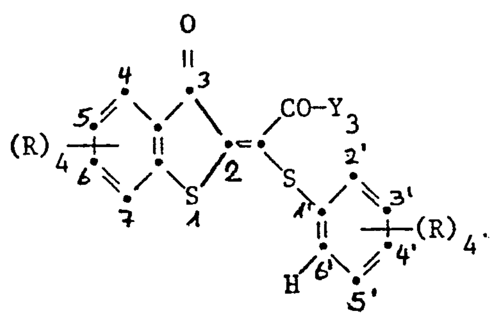

(Va),

worin zwei R Wasserstoff oder Alkyl mit 1-12 C-Atomen, ein R Wasserstoff, ein Halogenatom oder Alkyl mit 1-12 C-Atomen und ein R Wasserstoff, ein Halogenatom, Alkyl, Alkoxy, Alkylthio, Alkoxyalkyl, Alkanoyloxyalkyl oder Alkylcarbamoyl mit je 1-12 C-Atomen in den Alkyl- und Alkoxyteilen und 2-13 C-Atomen in den Alkanoylteilen oder zwei benachbarte Substituenten R zusammen Trimethylen oder Tetramethylen und die restlichen R Wasserstoff und $Y_3$ ein Halogenatom, -OH, $C_{1-6}$-Alkoxy, Phenoxy oder ein Alkalimetallkation bedeuten.

0023017

Europäisches Patentamt

# EUROPÄISCHER RECHERCHENBERICHT

Nummer der Anmeldung
EP 80 10 4106

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | GB - A - 2 022 606 (BAYER)<br>* Ansprüche 1-14 *<br>-- | 1 |
| A | US - A - 2 539 737 (F. GRIESHABER)<br>* Ansprüche; Seite 1, Spalte 2, Zeile 40 - Seite 2, Spalte 1, Zeile 12 *<br>-- | 1 |
| A | FR - A - 823 196 (SOC. POUR L'INDUSTRIE CHIM. BÂLE)<br>* Zusammenfassung; Seite 2, Beispiel 1 *<br>-- | 1 |
| A | FR - A - 390 484 (FARBWERKE MEISTER LUCIUS & BRÜNING)<br>* Zusammenfassung *<br>-- | 1 |
| D,A | US - A - 2 804 464 (E. KAPLAN)<br>* Ansprüche 1-9 *<br>-- | 1 |
| A | FR - A - 385 044 (BASF)<br>* Zusammenfassung *<br>& DE - C - 205 324<br>----- | 1 |

KLASSIFIKATION DER ANMELDUNG (Int Cl ³)

C 09 B 7/10
C 07 D 333/64
333/74
333/78
C 07 C 149/20

RECHERCHIERTE SACHGEBIETE (Int Cl ³)

C 09 B 7/10
7/12
C 07 D 333/64
333/74
333/78
C 07 C 149/20

KATEGORIE DER GENANNTEN DOKUMENTE

- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 16-10-1980 | DELANGHE |